# EUROPEAN PATENT SPECIFICATION

(11) **EP 1 363 652 B1**
(45) Date of publication and mention of the grant of the patent: **17.05.2006**
(21) Application number: 02715994.6
(22) Date of filing: 30.01.2002
(51) Int. Cl.: A61K 36/74, A61K 31/49, A61K 31/19, A61P 25/24, A61P 25/32

(54) **Extract of green coffee beans, pharmaceutical composition containing this extract, use for the treatment of depression and alcoholism as well as its process of preparation**
Extrakt aus grünen Kaffeebohnen, pharmazeutische Zubereitung enthaltend diesen Extrakt, Verwendung zur Behandlung von Depressionen und Akloholismus sowie Herstellungsverfahren
Extrait des grains de café vert, produits pharmaceutiques contenant ce extrait, utilisation pour le traitment de la depression et de l'alcoolisme ainsi que le procédé de preparation

(30) Priority: 30.01.2001 BR 0100444
(43) Date of publication of application: 26.11.2003
(73) Proprietor: Biosintética Farmacêutica Ltda, 04795-000 Jurubatuba, São Paulo SP (BR); Fundação José Pelúcio Ferreira, Centro - Rio de Janeiro - RJ (BE)
(72) Inventor: TRUGO, Luis, Carlos University Professor, 22039-010 Rio de Janeiro, RJ (BR); LIMA, Darcy Roberto Andrade, 21020-170 Petropolis, RJ (BR)
(74) Representative: Minoja, Fabrizio
(86) International application number: PCT/BR2002/000014
(87) International publication number: WO 2002/060462

(56) References cited:
- WO-A-86/01508
- KAWACHI, I. ET AL.: "A prospective study of coffee drinking and suicide in women" ARCHIVES OF INTERNAL MEDICINE, vol. 156, 11 March 1996 (1996-03-11), pages 521-525, XP008007071 cited in the application
- PATENT ABSTRACTS OF JAPAN vol. 1996, no. 08, 30 August 1996 (1996-08-30) & JP 08 092057 A (ICHIMARU PHARCOS CO LTD;U C C UESHIMA CAFE KK; ORIZA YUKA KK), 9 April 1996 (1996-04-09)

## Description

The present invention relates to the process to obtain a semi-synthetic, prepared coffee extract, in which the coffee natural composition is modified / concentrated, with caffeine content within the range from 80 to 90 mg, and chlorogenic acids content within the range from 250 to 450 mg per 1200 mg of dry extract (± 5% moisture), and [to obtain] pharmaceutical products, capsules and suspension, both for oral use, obtained from it, to be indicated for the prophylactic treatment of depression and alcoholism.

### DEPRESSION

Studies by one of the inventors (Darcy Lima), confirmed by international studies by major research centers in the United States (KAISER PERMANENT MEDICAL CENTER and HARVARD UNIVERSITY) have evidenced that mental depression is a normal response, and even necessary, of the human brain, aiming at the individual's social adaptation. It is, in fact, a highly evolved reaction form of the human brain in the animal scale, counterbalancing the effects of the man individualist excesses, which disturb his social integration, and often lead him to break established rules. Only the depressive response tends to integrate the individual in a society, to valorize it and to adapt to it.

Thus, every human being periodically shows mental depression, as a normal manifestation of the brain. When it comes up without a triggering reason, is too intense or lasts too long, the individual might need help.

About 20% of the adult population shows, during their lives, depressive episodes with significant clinical manifestations, which require specialized treatment, with medications. It is about this depression, named pathologic, we are going to talk about next.

Depression is the most common psychiatric disturbance in adults. An almost universal, no matter the country, observation, is its higher incidence on women than on men; it may start from childhood to old age, but 50% of the patients are between 20 and 50 years old, with the average age of about 40 years old; race and social-economical condition are not related to depression prevalence. In the United States, depression affects 11 million people, with yearly losses of 43.7 billion dollars, including absences from work, productivity drop, salaries, medical treatment and expenses with suicide cases. In Brazil, 10 million people are estimated to be afflicted by the disease.

The causes of depression are not totally known, but three groups of factors are especially involved in its etiology:

### • Biological factors

Neurochemical - The noradrenaline and the serotonin would be the two most involved neurotransmitters in the depressive disturbance physiopathology. Experimental and clinical data indicate that the dopaminergic activity would be reduced in depression.

In addition, and even though the data are not conclusive up to the present moment, the neurotransmitter amino acids (especially GABA) and the neuroactive peptides (especially vasopressin and the endogenous opioids) have been involved in the physiopathology of some mood disturbances.

Neuroendocrine regulation - Several neuroendocrine malfunctions have been reported in patients with mood disturbances. Abnormalities of the limbic - hypothalamic - pituitary - adrenal (LHPA) axis are the most consistently reported ones. The finding that the cortisol hypersecretion is present in some depressed patients has been used in the dexamethasone suppression test (TSD). This one, the dexamethasone, is an exogenous steroid that suppresses the cortisol blood level. The TSD is abnormal in about 50% of the depressed patients, indicating an HLPA axis hyperactivity. The TSD, however, is not specific for depression, as it may be abnormal in patients with organic brain disturbances (e.g., Alzheimer's disease) and other medical conditions.

### • Genetic factors

The fact that depressive disturbance occurs in families is also compatible with a biological etiology for the human disturbances. The evidence of heredity for the bipolar disturbance (successively alternating depression and mania episodes) is stronger than for the classic depressive disturbance (unipolar depression), about which we have been talking. If one of the parents has bipolar disturbance, there is a 27% chance that any child has a mood disturbance; if both parents have bipolar disturbance, there is from 50 to 75% chance of a child to have a mood disturbance. Studies with twins have been showing an agreement rate of 67% for bipolar disturbance in monozygotic twins and 20% for the same disturbance in dizygotic twins. The genetic data, therefore, are compatible with the conception of a genetic basis for the depressive disturbances.

### • Psychosocial factors

Paramount events and environmental stress - Some doctors believe the paramount events play a primary role in depression; others are more conservative, suggesting that the paramount events play an only limited role at the beginning and temporal situation of depression. The most convincing data indicate that the paramount event which is most associated to the later development of depression is the loss of one of the parents before 11 years old. The environmental stresser which is most associated to the beginning of a depression episode is the loss of a spouse.

Pre-morbid personality factors - No isolated feature or kind of personality has been established as being solely predisposing to depression. All the human beings, with any personality standard, may and get depressed under appropriate circumstances; however, certain kinds of personality - oral-dependent, obsessive-compulsive, and hysteric - may show a higher disposition to depression than the anti-social, paranoid personality kinds, and others who use projection and other externalizing defense mechanisms.

A depressed mood and a loss of interest or pleasure are the key symptoms of depression. The patients may say they feel discouraged, hopeless, miserable, or useless. For the patient, the depressed mood has often a distinct quality, which differentiates it from the completely normal sadness emotion. The patients often describe the depression symptoms as an agonizing emotional pain.

About two thirds of depressed patients think of suicide, and 10 to 15% commit it. The depressed patients sometimes complain about not being able to cry, a symptom that is relieved as they get better. The depressed patients, however, sometimes seem not to be conscious of their depression, and they do not complain of a mood disturbance, although they may show withdrawing from family, friends and activities in which they were previously interested.

Almost all the depressed patients (97%) complain about reduced energy, resulting in difficulties to complete tasks, school and work losses, and less motivation to carry on new projects. About 90% of the patients complain about sleep disturbances, especially early awaking (i.e., terminal insomnia) and multiple awakenings at night, during which they wonder about their problems.

Many patients have appetite decrease and weight loss. Some, however, have appetite increase, weight gain and increased sleep. These latter ones, especially when their symptoms are accompanied by strong anxiety, are sometimes classified as showing atypical depression, but this term has acquired many different meanings, and is not much practically useful anymore. Anxiety is actually a common depression symptom, affecting up to 90% of the depressed patients. The several changes in food consumption and rest may worsen coexisting medical diseases, such as diabetes, hypertension, chronic obstructive lung disease and cardiopathy. Other vegetative symptoms include abnormal menstruation and decreased sexual interest / performance. Sexual problems may, sometimes, lead to inappropriate sending, such as conjugal counseling or sexual therapy, if the doctor does not recognize the underlying depressive disturbance.

Anxiety (including panic attacks), alcohol abuse and somatic complaints (such as constipation and headache) often complicate the treatment of depression. About 50% of all the patients describe a daily variation in their symptoms, worst in the morning and symptoms decrease at night. The cognitive symptoms include subjective reports of incapacity to concentrate (84% of the patients) and thought compromising (67%).

### Treatment of depression

The state of the technique does not comprise a safe and effective prophylactic treatment method for depression; the existing treatment methods are addressed to the already installed disease.

The first, main and most critical decision the doctor shall take is about hospitalizing the patient or trying the external treatment. Clear indications for hospitalization are the need of diagnostic procedures, suicide or homicide risk and broadly reduced capacity, from the patient, to take care of himself (feeding, sheltering and dressing). A history of fast progression symptoms and rupture of the usual supporting systems are also indications for hospitalization. Mild depression can surely be treated at the office, if the doctor often evaluates the patient. The clinical signs of impaired judgement, weight loss or insomnia shall be minimum. The patient supporting system must be strong, neither overdeveloped nor distant. Any significant alterations in the symptoms, external behavior and supporting system attitude are enough to justify the hospitalization.

The acute depression is a treatable condition in 70 to 80% of the patients. Most studies have showed that a combination of psychotherapy (interpersonal, cognitive, psychoanalytic or behavioral) and pharmacotherapy is the most effective method to treat depression.

The so-called antidepressant drugs, available in the market for use in the pharmacological treatment of depression, are classified in groups, considering their structure or central neurotransmitter on which they exert their actions. To the oldest antidepressants, the cyclical (tricyclic and tetracyclic) and the monoamine-oxidase (MAOIs) inhibitors, joint later the selective serotonin recapture inhibitors (SSRIs), the type A revertible inhibitors of monoamine-oxidase (RIMAs), and more recently, the serotonin and noradrenaline recapture inhibitors (SNRIs). Other antidepressants which do not fit exactly in these groups include bupropione, mirtazapine, nefazodone, reboxetin, trazodone and viloxazine.

Lithium (900 to 1200 mg daily, serum level between 0.6 and 0.8 mEq/l) may be added to the treatment with an antidepressant for 7 to 14 days. According to some observations, this procedure would turn a significant number of individuals who do not respond to the antidepressant into people who respond to this treatment. The lithium would potentiate the serotonergic neuronal system, thus increasing the antidepressant effectiveness range.

The tricyclic antidepressants have been preferred to the MAOIs, for the treatment of depressive episodes, due to the problem of interactions with other drugs and the need of strict dietary precautions involving the MAOIs use. Unfortunately, the traditional tricyclics, such as the amitriptyline, are associated to adverse effects, which may limit their use or cause suffering to the patient, and because, due to its cardiotoxicity, they are associated to a high fatality risk in patients who take overdoses upon suicide attempts. The urge, in the last years, for new antidepressants, has improved the patients treatment options. Several drugs related to the tricyclics group, such as the lofepramine and the mianserin are less cardiotoxic than the first tricyclics. Subsequently, selective serotonin recapture inhibitors (SSRIs), such as the fluoxetine, were developed and brought other important improvements in the adverse effects and safety profile of this class of products. More recently, serotonin and noradrenaline recapture inhibitors (SNRIs), such as the venlafaxine, have been developed, as well as revertible inhibitors of type A monoamine-oxidase (RIMAs), such as the moclobemide.

Antidepressants as nefazodone, mirtazapine and reboxetin, which have slightly different biochemical profiles from those of the larger groups, have also been introduced in the market recently.

Even though such progresses are welcome, they haven't been accompanied by any significant improvement in the antidepressant activity. This has been leading to much debate about the choice of the antidepressant therapy, mainly about the option between a tricyclic or a SSRI. There are arguments supporting the use of SSRIs as first line, due to the rarer unpleasant side effects, although they may cause a different series of adverse effects that may be a problem in some patients; the same arguments may also be applied to the SNRIs, RIMAs and other new antidepressants. However, the tricyclics remain the first line preferred by numerous professionals, due to the wide experience with their use and familiarity with their pharmacological actions.

The tricyclics with sedative properties may be more appropriate for restless and anxious patients, while those with less sedative properties may be preferred for withdrawn and apathetic patients. The tricyclics cause antimuscarinic and cardiotoxic side effects in several degrees, and these properties make their use impossible in some patients, to whom the SSRIs may be a safer choice. However, the SSRIs themselves cause characteristic side effects; gastrointestinal symptoms, such as nausea and vomits, may be a problem, and sleep disturbances and anxiety may be exacerbated at the beginning of the treatment. In addition, the SSRIs in current clinical use interact in several degrees with the P450 cytochrome enzymatic system, what carries along a potential of interaction with a wide range of substances.

The MAOIs are seldom used as first line antidepressants, but they are particularly effective in atypical depressions. The RIMAs are a safe alternative to the MAOIs and less dietary restrictions are required.

The sedative properties of some antidepressants may adversely affect the patients performance in some potentially dangerous activities, such as driving vehicles and operating machines. In these situations, whenever possible, a less sedative antidepressant should be preferred, although, concerning this matter, caution is recommended with all the psychotropic drugs, especially at the beginning of the treatment.

An area of special interest, concerning the initial choice of the antidepressant is which drug is the most appropriate for patients considered high suicide risks, to whom the occurrence of overdose due to intentional ingestion of the drug is an always present possibility: the first tricyclics and the maprotiline seem to be more toxic in overdose than mianserin and the SSRIs; the MAOIs show an intermediate risk. Within the tricyclics group, the desipramine has been reported as more often associated to fatal overdose, while lofepramine may be one of the safest tricyclics. Although such analyses can not determine to what extent the data reflect prescription standards and patients selection, unlike the drug toxicity, it is widely accepted that the old tricyclics are toxic in overdose and that the safest options are the SSRIs and the newest tricyclics and cyclical-related. In practice, it is often hard to identify patients with high suicide risk and, therefore, it has been suggested that a routine strategy should be starting the antidepressant therapy in all the patients with drugs having low toxicity in overdose. However, whatever the chosen antidepressant is, all the patients must be carefully monitored at the beginning of the treatment, until an improvement is observed in depression and smaller quantities of the antidepressant may be prescribed.

### ALCOHOLISM

Alcoholism used to be a generally used term for a disturbance marked by the chronic and excessive use of alcohol, a psychoactive substance (in the organism, it may alter conscience or the cerebral condition), resulting in psychological, interpersonal and medical problems. Nowadays, the denomination alcohol dependence is preferred for this disturbance, characterized by any of these three main patterns of pathological use of the substance: 1) need to use big quantities of alcohol daily for the appropriate functioning; 2) irregular and heavy ingestion of alcoholic drinks, limited to the weekends, and 3) long sobriety periods intercalated with periods of intense drinking, which last weeks or months. These patterns involve behaviors as: 1) incapacity to reduce drinking or to stop drinking; 2) repeated efforts to control or reduce the alcoholic ingestion to certain times of the day; 3) booze-ups (remain intoxicated all day long, for at least two days); 4) occasional consumption of 750 ml of distilled drinks (or its equivalent in wine or beer); 5) amnesiac periods for events occurring during the intoxication (blackouts); 6) continue the ingestion of alcoholic drinks, despite a serious disturbance the individual knows to be exacerbated by the use of alcohol; and 7) ingestion of alcohol which is not appropriate for consumption, as for example, fuel, commercial products containing alcohol. In addition, the dependents show a social or occupational functioning compromised by the use of alcohol, such as violence when intoxicated, absences from work, getting fired, legal difficulties (for example, arrestment due to intoxicated behavior, traffic accidents while intoxicated) and discussions or difficulties with the family or friends due to the excessive use of alcohol.

Alcohol is the main psychoactive drug used all over the world. In the United States, it is estimated that there are about 13 million dependent people, or who use it abusively (an only less severe kind of the disturbance, and more, to some authors, one of its possible initial configurations, involving a pattern of adverse repercussions of the repeated use, which does not satisfy substance dependence criteria). After heart diseases and cancer, alcoholism is the third biggest health problem in the United State, nowadays. According to the National Institute of Drug Abuse (NIDA), in a publication dated July, 1989: 1) 50% of the young people (from 12 to 17 years old) tasted an alcoholic drink at any moment in their lives, and 25% ingested alcoholic drinks at least once in the previous month; 2) among young adults (from 18 to 25 years old), the prevalence of alcohol ingestion is substantially superior to the young people from 12 to 17 years old: 90% tasted an alcoholic drink at any moment in their lives; 82% used alcohol in the previous year, and 65% used alcohol during the previous month; 3) the number of alcohol users, in 1988, was 106 million people (in 1985 it was 113 million); 4) from the 135 million people who ingested alcohol in the previous year, more than one third, about 47 million people, drank once a week, or more often.

The alcoholism is associated to, at least, 50% of accidents with death, 50% of the homicides and 25% of the suicides. In addition to the harmful consequences on the personal and professional relationships, the habitual user of alcoholic drinks is a potential candidate to problems as gastritis, hepatic cirrhosis, myocardiopathy, alcoholic hallucinosis and/or Korsakoff amnesiac syndrome. Alcoholism reduces life expectation in about 10 years, and leads all the other drugs concerning deaths related to drugs.

In the year of 1990, in the United States, more than 136 billion dollars were spent with problems resulting, direct or indirectly, from alcohol consumption, such as users treatment, accidents, violence and productivity loss. In Brazil, this loss is equivalent to more than 5% of the PIB [Gross National Product], over 15 billion dollars yearly.

Even though the causes of alcoholism are not totally known, some groups of factors are outstanding in its etiology:

### • Biological factors

The ethanol (ethyl alcohol) exerts effects on the gamma-amino-butiric acid (GABA) neurotransmitter system, what would explain the cross tolerance and the addictive effects that may occur when alcohol is used in combination with barbituric drugs and benzodiazepinic drugs, both of which act on the GABA system. Ethanol has also showed relatively specific effects on the noradrenergic neurons in the locus ceruleus and on the dopaminergic neurons in the ventral tegmental area.

### • Genetic factors

The genetic component is very important for the alcoholism development. Alcoholics children become alcoholics too four times more often than the non-alcoholics children. The sons of alcoholics are more vulnerable to alcoholism than the daughters. In a longitudinal study carried out in Sweden along 30 years, in adopted children, who in the future became alcoholics, about 25% had parents who also were alcoholics. Another Swedish study found out that monozygotic twins showed an agreement rate twice as higher for alcoholism than the dizygotic twins of the same sex. Other studies report a higher agreement for the alcoholism among dizygotic twins than between non-twins.

### • Psychological and Psychosocial factors

An infantile history of attention deficit disturbance with hyperactivity, or behavior disturbance, or both, increases the risk of the child becoming an alcoholic, especially if there is alcoholism in the family.

Alcoholism tends particularly to coexist with a mood disturbance diagnosis. Several studies estimate the prevalence of depression for all the life in alcoholic people, within the range from 10 to 50%. The personality disturbances, particularly the anti-social personality disturbance, may also predispose to alcoholism.

Alcohol is extremely effective for relieving anxiety, and many people use it for this purpose.

### Alcoholism treatment

The state of the technique does not comprise a safe and effective prophylactic treatment for the alcoholism. The already installed disease, and not complicated by alcohol-induced organic cerebral condition, is treated in a multiple treatment approach: psychotherapy, medicines, behavioral therapy, anonymous alcoholics (AA).

Two groups of drugs are used for the treatment of alcoholism: disulphiram, an aversive drug and psychotropic drugs. Recently, FDA (USA) has approved the use of naltrexone, an opioid antagonist, for the treatment of alcoholism.

### Disulphiram

Disulphiram (Antietanol) competitively inhibits the aldehyde desidrogenase enzyme, in such a way that even a single dose of alcoholic drink generally causes a toxic reaction, due to the accumulation of cetyl-aldehyde in blood. The drug administration shall not start before 24 hours have elapsed since the last ingestion of alcohol. The patient must be in good shape, highly motivated, and be cooperative. The doctor shall warn him about the consequences of alcohol ingestion while he is taking the drug or up to 2 weeks after its discontinuation. People who ingest alcohol while taking 250 mg disulphiram daily experience blushing and heat sensation on the face, sclerotic, upper limbs and thorax; may get pale, hypotensive and nauseated, experiencing serious discomfort, and dizziness, turbid vision, palpitations, shortness of breath and dormancy in extremities may also occur. The most serious potential consequence is severe hypotension. This same response may be showed by the patients when ingesting alcohol in products as vinegar, or after inhalation of after-shave lotions or perfumes. Once triggered, the described syndrome lasts, typically, from 30 to 60 minutes, sometimes a little longer. With disulphiram doses over 250 mg toxic psychoses may occur, with memory compromising and mental confusion. The drug may also exacerbate psychotic symptoms in schizophrenic patients.

### Psychotropics

The anxiolytic agents and the antidepressant are useful during several steps of the treatment. At the initial step, the abstinence one, anxiety, restlessness and insomnia are prominent, and they may be controlled with agents as diazepam and chlordiazeproxide. The antidepressants are useful for the clinically depressed patients while they abstain.

Most alcoholic patients search treatment as a result of the pressure exerted by the spouse, employer, or by the fear that the perpetuation of the drinking habit drive them to a fatal consequence. The patients who are persuaded, encouraged or even coerced to the treatment by people who are important to them (wife, children) are more capable of remaining in treatment, and they show a better prognosis than the ones who are not pressured. The best prognosis is for people who look for a psychiatrist voluntarily, because they conclude they are sick and need help.

Generally, and as it occurs with all the other drugs used abusively (nicotine, marijuana, cocaine, heroin, etc.), the user recovery is considered difficult, in such a way that most specialists do not believe that there is an "ex-addicted" condition, but only controlled "addicted people".

### Naltrexone

Naltrexone, an opioid antagonist pharmaco, has been recently approved by FDA (USA) for the alcoholism treatment. In the 50 mg daily dose, or in alternate days, the pharmaco blocks the craving of consuming alcohol, as well as diminishes the number of days with alcohol consumption. The main side effect resulting from its use is nausea (10% of the patients), which may lead to abandoning the treatment. This is carried out for the period of 12 weeks, when its use is interrupted, and it is fundamental to associate a psychotherapy, as well as following-up the patient, and including him in support groups as the AA. High doses of naltrexone (over 300 mg/day) may be hepatotoxic. Its safety for young people under 18 has not been established yet.

### ILLICIT DRUGS

Dependence on illicit chemical substances (drugs) is nowadays a growing and alarming source of medical and social concern. The planting, processing and traffic of plants which originate drugs as marijuana, cocaine and heroin, as well as of the ready-to-use drugs, is one of the most important business nowadays, and unquestionably the most profitable one all over the world. The resources are around 500 billion dollars yearly, an amount corresponding to twice as much all the American currency. In the American market, the biggest one for drugs, this business produces yearly profits of about 100 billion dollars, twice as much what is spent there with petrol. And, despite all the efforts exerted to combat it, it is a permanently expanding business. It is estimated that more than 30 million Americans smoke marijuana (25% of the American young people), more than 8 million use cocaine regularly (6% of all the young people and 12% among the ones from 18 to 21 years old), and more than 500,000 are addicted to heroin. Studies carried out in Brazil have been evidencing that, likewise, this threaten is important and growing in our Country.

The severe harmful consequences are well known, for the individual, the family and the society, of the dependence or abusive use of these drugs, which, many times, incapacitate people and leads them to crime, with no effective prophylactic (or healing) known means, in the state of the technique, to face them. In addition, there is also the fact that, recently, the injectable drugs consumption got associated to the dissemination of a potentially fatal disease, AIDS (acquired immunodeficiency syndrome): in the United States, it is estimated that the transmission of 1/3 of all the AIDS cases has happened by means of contaminated syringes; more than 50 countries have already reported the virus transmission among people who use illicit drugs in the injectable form.

As it happens in alcoholism, concerning the etiology, one cause is not determined for the addiction to drugs, but a set of biological, genetic, psychological and social factors determining the addictive behavior. Definite and very important is the existence of what we could call a psychological and behavioral profile of adolescent addicted people (adolescence, when, most of times, the habit starts) to drugs in general, licit or illicit: underlying depression, often of the restless type, and accompanied by anxiety symptoms; impulsiveness expressed by a passive-aggressive orientation; fear of failure; use of drug as an anti-anxiety agent, in order to mask feelings of low self-esteem, hopelessness and aggressiveness; limited strategies of confrontation and low tolerance to frustration, accompanied by the need of immediate rewarding; susceptibility to the drug contingencies, with a sharp perception of the relation between the good sensations and the act of taking the drug; feelings of behavioral impotence counterbalanced by the momentary control over the life situation by means of the drugs; disturbances in the social and interpersonal relationships, with the mates maintained by mutual experiences with drugs. The authentic engagement, not being attractive or possible for many, the drugs abuse becomes, for a growing portion, a way of identification and involvement.

In the biological plan, and complementing what has already been said previously for the ALCOHOLISM, it shall be said that the cerebral chemistry that leads to the compulsive use of drugs and to the loss of control, with dependence development, is the result of the use of drugs as cocaine, amphetamins, opiates, nicotine and alcohol, by a vulnerable individual (here, we mean psycho-emotionally vulnerable), in an appropriate dose, in an appropriate frequency and in an appropriate time. The excessive brain bombardment by these substances causes permanent molecular adaptations in three neuronal systems classes: 1) systems involved in the autonomic control and other somatic functions, leading to physical dependence; 2) systems acting on the motivation and behavior control, leading to a loss of control; and 3) systems that produce powerful association memory impulses, which predispose and induce to searching drugs and to relapse, even in the individual who has been disintoxicated.

All the drugs that cause dependence seem to activate and produce permanent alterations in the dopaminergic circuits (also involved in the depressive conditions, as we have already seen), which control motivation and behavior. It is believed that the main way involved in the origin of dependence to drugs seems to be the dopaminergic way, which extends from the ventral tegmental area (VTA), a mesencephalic reticular formation region that sends fibers to the telencephalic areas belonging to the limbic system, as the amygdaloid nucleus, the septal area and the surcingle turn cortex. The pre-frontal cortex and the corpus striatum also receive fibers there originated. Also from the mesencephalon depart fibers to the base nuclei, more specifically to the ventral striated, basically to the acumbens nucleus, a mass of grayish substance located ventrally to the nucleus caudatus head, which has connections with the limbic system. This mesolimbic way seem to be involved also in motivation and behavior necessary for human survival, and for the reproductive process, including the reproduction act itself. For example, the food choice and consumption process may not have been selected in a specific way in the evolutionary process. But, by means of this dopaminergic mesolimbic system activation, food (and drugs) may lead to reward and conditioning, strengthening memory and learning mechanisms.

When something stimulates this system, it is immediately recognized and vividly remembered, including the circumstances that lead to its use or consumption.

Nicotine, ethanol, cocaine and the opioids are all originally plants or natural fermentation byproducts, and act as conditioning substances, able to generate dependence, as they mimic or increase the actions of the neurotransmitters that act on the reward, pleasure and learning mechanisms of the human being. Cocaine inhibits the dopamine recapture, increasing the duration of its action and its effects on the mesolimbic system synapses, while amphetamin increases the dopaminergic neurons release of dopamine. Opioids like morphine and heroin mimic opioid neurotransmitters (encephalins), which act directly on the acumbens nucleus, but can also, acting in a uninhibitory way on the ventral tegmental area (VTA), favor the dopamine release. Nicotine mimics the acethylcoline action on the central nicotinic receptors, while ethanol exerts powerful facilitating effect on the GABAergic (GABAa) receptors.

Although the nicotine and the ethanol actions on the brain reward circuits are not completely known yet, it is known that both substances cause a greater dopamine release on the acumbens nucleus and limbic system. And this increase in the dopamine levels may be inhibited in several steps, as through the use of the system opioid receptors antagonists, as the naltrexone, which constitutes a new advance in the alcoholism treatment, or of the bupropion antidepressant, an agent that has been recently approved by the FDA (Food and Drug Administration, USA), for tobaccoism control.

Recent discoveries in medicine have evidenced that the human being, in addition to selecting plants (nicotine, cocaine, heroin, etc.), which provoke, via the limbic system, the pleasure sensation, has also identified plants, such as coffee, which are able to block this opioid system, modulating or interfering in the desire of self-rewarding, which leads to drugs consumption.

Coffee is the most consumed plant by the human species, and its trade constitutes the second highest in the planet, surpassed only by petrol. Brazil is the main coffee producer, responsible for about one third of the world production, and it has already been over 70% of total some decades ago. This trade generates foreign exchange credits around two and a half billion dollars (US$ 2,500,000,000) yearly to the country.

Coffee consumption started with an Arabian shepherd named Kaldi, in Ethiopia, around the seventh century, who observed the stimulating effects of green coffee grains on his goats. When he ingested the grains, he felt the same stimulating effects. The effects of this plant soon spread and many started ingesting it. As the fruits dried when transported, the shepherds decided to prepare a beverage with them. And the fruits were put in hot water, in order to help combating cold in the vigil nights. Thus was created the beverage that started to be called KAHWAH or CAHUE, which means "STRENGTH". The history continues with Mohammed's actuation, who stimulated his priests to boil the little fruits in water and drink the resulting liquid, for everybody to be able to remain awake during the night to pray on behalf of the prophet.

Around the year 900, an Arabic medical book suggested the use of coffee for almost all the medical problems, such as sexual apathy, fevers in general, including measles. This fact, in addition to the Islamism expansion, made the coffee consumption vertiginously increase along the years, but restricted to the Orient. After the Arabic world, coffee started to be consumed also by the Turkish, who thus became more awake and active for the fights and for the Turkish-Ottoman Empire expansion, up to the conquest of Constantinople, in 1453.

Due to being a beverage consumed by people considered pagans by the Christianism, the beverage was vehemently avoided in the Occidental world. Only by the end of the sixteenth century its regular consumption was permitted among Christians, after pope Clemens VIII (1592 - 1605) tasted and liked coffee, which stimulated his intellectual capacity and improved the mood, allowing him to stay up late to dedicate to prays to God. The pope advised Christians to do the same, and the coffee consumption increased quickly in the Occidental world. Introduced in the New World by the Oriental Indians Company, coffee became one of the main agricultural products of that time. And as its cultivation was difficult in England and France, due to the cold weather, it developed more in tropical countries. From the sixteenth century on, the coffee consumption in France and England started to be an option, and even an alternative against the abusive consumption of alcoholic drinks, such as wine, whiskey, rum, beer and others, to the time it starts being a victim of prejudicial campaigns, as something harmful for health.

At the beginning of the Third Millennium, mankind faces a serious threaten to its physical and mental health: depression (with its several possible consequences, including the abusive consumption of alcohol and other drugs., and the dependence on licit and illicit drugs. In addition, legal (tobacco) and illegal (marijuana, cocaine) toxic plants are substituting coffee planting in several countries, such as Bolivia, Peru, Colombia and even Brazil. In 1987, coffee importation by the United States was about 3.13 billion dollars, with a reduction of almost 15%, when compared to the 3.87 billion dollars importation in 1980. The per capita consumption has also decreased, from 3.8 kg in 1983 to 3.6 kg in 1985. Two factors may be held responsible for this important drop in the beverage consumption: 1) the negative propaganda against coffee (supported by competing economical interests), accused of being a harmful beverage for the consumer health, due to containing caffeine; the absence of research in the producer countries, either to contest the groundless accusations against coffee consumption, or to investigate and find out benefic aspects consequential to the consumption of this plant.

And while the coffee per capita consumption decreased in most countries, the incidence of depression, alcoholism and addiction to illicit drugs increases.

Recent studies show that the moderate consumption of the beverage is not harmful, on the contrary, it is benefic for the human health, especially for the prevention of problems, such as depression / suicide and alcoholism / cirrhosis, among others. It is possible to say, unquestionably, that, in appropriate quantities (up to 500 mg daily), equivalent to half a liter or four big cups divided during the day, caffeine is not harmful for the normal and healthy human being, neither is a triggering agent of any kind of disease, on the contrary, it may be a benefic substance for the human organism, especially the brain.

Coffee, in addition to caffeine (± 2.5%), which has central stimulating action, has a bigger quantity (± 7%) of chlorogenic acids, with opioid antagonist effect. Coffee, in addition to its strict medicinal value, result of the presence of those two substances in its composition, is actually a rich food. It has, in addition to a great variety of minerals, such as potassium (k), magnesium (Mg), calcium (Ca), sodium (Na), iron (Fe), manganese (Mn), rubidium (Rb), zinc (Zn), copper (Cu), strontium (Ss), chrome (Cr), vanadium (V), barium (Ba), nickel (Ni), cobalt (Co), lead (Pb), molybdenum (Mo), titanium (Ti) and cadmium (Cd); amino acids such as alanine, arginine, asparagine, cysteine, glutamic acid, glycine, histidine, isoleucine, lysine, methionine, phenylalanine, proline, serine, treonine, tyrosine and valine; lipids, such as triglycerides and free fatty acids ; sugars, such as sucrose, glucose, fructose, arabinose, galactose, maltose and polysaccharides. Additionally, coffee has also a vitamin from the B complex, niacin (PP vitamin - Pelagra Preventing).

Coffee is a plant (Coffea arabica and Coffea robusta, the species cultivated in Brazil) consumed, mainly, in the form of a beverage resulting from infusion or decoction of the so-called "coffee powder", and its passage through a paper or cloth filter. The "coffee powder" is obtained with the green grains roasting, at a temperature varying from 200°C to 500°C, during 5 to 15 minutes, followed by its grinding. Among its components, only caffeine is thermostable, i.e., it is not destroyed by excessive roasting. Other substances present in its composition, such as amino acids, sugars, lipids, niacin and the chlorogenic acids, for not being thermostable, will be preserved or destroyed during the roasting process, depending on its characteristics. See chart below.

**GREEN COFFEE AND ROASTED COFFEE COMPOSITION**

| Substance | GREEN COFFEE | | ROASTED COFFEE |
|---|---|---|---|
| | Coffea arabica | Coffea robusta | |
| Caffeine | 1-1.5% | 3.0 - 4.5% | Thermostable |
| Trigonelline | 1% | 0.75% | Depends on the roasting |
| Niacin (PP vitamin) | 0.5% | 0.5% | Depends on the roasting |
| Chlorogenic Acids | 5 - 7% | 7 -9% | Depends on the roasting |
| Amino Acids | 2.0% | 2.5% | Depends on the roasting |
| Mineral Salts | 3 - 4% | 4 - 5% | Depends on the roasting |
| Sugars | 50 - 55% | 35 - 45% | Depends on the roasting |
| Lipids | 10 - 20% | 10 - 15% | Depends on the roasting |
| Others: ashes, pigments, oils, cafestol, etc. | 20 - 30% | 25 - 45% | Depends on the roasting |

What interests us here are the caffeine and the chlorogenic acids, substances responsible for the actions of this extract with antidepressant and opioid antagonist properties at the SNC (Central Nervous System) level, which make it effective for the prophylactic treatment of depression and alcoholism.

### Caffeine

Caffeine is a methylxanthine, which, as the teophilin, exerts several actions: it inhibits the phosphodiesterase enzyme and exerts an antagonist effect on the central receptors of adenosine. It is a powerful stimulator of the SNC (Central Nervous System), especially of the upper centers, producing an increased vigilance and mental activity state (less somnolence and fatigue, a faster and clearer thought flow, increased capacity of intellectual effort and decreased reaction time). It is not a powerful bronchodilator, but, by stimulating the respiratory center, it increases breathing speed and depth. Its stimulating action on the medullar vasomotor center and its positive inotropic action on the myocardium are compensated by its peripheral vasodilator effect on the arterioles, in such a way that it exerts, generally, little effect on the blood pressure. Caffeine facilitates the execution of muscular work and increases the total work that may be made by a muscle. Finally, it has some diuretic action.

Thus, daily ingestion of a moderate quantity of caffeine (up to 500 mg daily, what corresponds to about 4 big coffee cups daily) is benefic for the individual, man or woman, young or adult, who does not suffer from a severe mental disease, renal or hepatic failure and cardiopathies.

### Chlorogenic acids

In addition to the caffeine, which stimulates the cerebral cortex, the Coffea ssp. (coffee) plant has, in a bigger quantity, chologenic acids (5 - 9%), which, in the roasting process, and depending on its nature, form at least five derivatives from quinic acid: cafeoilquinic acid (CQA), dicafeoilquinic acid (diCQA), feruloilquinic acid (FQA), coumaroilquinic acid (CoQA) and cafeoilferuoilquinic acid (CFQA). At least one of these components, the feruloilquinic acid, has a powerful opioid antagonist effect.

### COFFEE X DEPRESSION AND/OR ALCOHOLISM

### • Ann. Epidemiol., 1993; 3:375-381:

Pioneer epidemiologic study coordinated by Dr. ARTHUR KLATSKY, from Kaiser Permanent Medical Center (KPMC), Oakland, CA, about the relation between coffee or tea consumption and mortality. In the period from January, 1978 to December, 1985, 128,934 people were accompanied, a group representing the population from that area, who informed essential data about health and life habits. When all the variants had been analyzed, the authors observed a statistically significant relation between the consumption of up to 4 cups of coffee daily, and a lower incidence of depression / suicide and alcoholism / cirrhosis on the studied population, in which 4501 deaths occurred within the study interval. The research indicated important details among the coffee consumers, in whom, in addition to a lower incidence of depression / suicide and alcoholism / cirrhosis, cases of death caused by traffic accidents were less frequent, what may be due to a lower consumption of alcohol, drug associated to more than 70% of the traffic accidents. The authors affirm that, although a consumption of coffee over 6 cups daily may increase the risk of cardiovascular problems, this does not occur if this consumption is limited to 4 cups daily.

### • Arch. Inter. Med., 1996; 156:521-525

The Harvard University Epidemiology Department has carried out a study of the cohort type named NURSE'S HEALTHY STUDY (NHS), started in 1976, in which 121,700 women (nurses) from 11 States were registered and accompanied periodically, by using a standardized questionnaire about life habits and health problems (heart diseases, cancer). In 1980, a semi-quantitative questionnaire about feeding was added to the research, under the guidance of Prof. Walter Willet, MD. A follow-up study for ten years (from 1980 to 1990), of 86,626 from the women, aged between 34 and 59 years old and with no cardiovascular disease or cancer, showed the occurrence of 56 cases of suicide, what was considered a very low incidence for the event (incidence of suicide among depressed patients is 15%, and of depression on the general population is 20%). The authors suggested a strong co-relation between a higher consumption of coffee and a lower incidence of depression and suicide among the women studied.

### • CAN COFFEE HELP FIGHTING THE DRUG PROBLEM?

Gladis B. Flores, Fernanda Andrade & Darcy R. Lima

A study aiming at prospectively determining the incidence of depressive feelings, ingestion of alcohol and drugs consumption among young people in Brazil, and if there is a relation between the daily ingestion of coffee and variants such as depressive symptoms and consume of alcohol, smoke, sodas and illegal drugs, has been coordinated by one of the authors of the present invention, Prof. Darcy R. Lima. A 10-year (1990 - 2000) follow-up study was carried out, in 106,502 students of both sexes, from 10 to 20 years old in 1990. Information about depressive symptoms and ingestion of alcohol, smoke and illegal drugs, as well as about the daily ingestion of coffee and sodas were collected by means of an adapted standardized questionnaire.

Results - Depressive feelings and depression were observed in an age-related incidence of 3 to 18%, on a daily basis (7 to 32% on a weekly basis), among young people from 10 to 20 years old. The same was observed concerning the regular ingestion of alcohol, on a weekly basis that may reach 72.5% (and up to 9.4% on a daily basis), among the oldest young people. The informed consumption of illegal drugs is low (up to 3%) in all ages, but showing a growing tendency with the age. The daily moderate ingestion of coffee (in the morning, after lunch and dinner) varies a lot among young people from country and urban areas (age-related consumption varying from 2 to 36%). But among the daily drinkers of an average of 3 cups of coffee, with or without milk, our preliminary data suggest that there is an inverted association between the ingestion of coffee and depression symptoms and ingestion of alcohol. Statistically significant data were not found concerning tobaccoism and addiction to illegal drugs, although a multiple adjustment to illegal drugs consumption and ingestion of coffee showed a weak residual benefit of coffee consumption to decrease smoke and drugs abuse among young people, which data should be kept aside, as the information concerning the ingestion of illicit drugs lacks reliability.

Based on the history data, on the knowledge about the neurochemical mechanisms involved in depression, alcoholism and addiction to drugs, and about the properties of caffeine and chlorogenic acids, and on the data obtained from the studies that tried to determine the relation between the coffee consumption X depression and alcoholism, the authors have developed a semi-synthetic, prepared product, object of this patent, in which the natural composition of coffee is concentrated / modified, with a caffeine content within the range from 80 to 90 mg and a chlorogenic acids content within the range from 250 to 450 mg per 1200 mg of dry extract (± 5% moisture), obtained as described next:

### Obtainment process

### 1. Selection of grains

Green coffee grains of the Coffea arabica and Coffea robusta variety, in equal quantities of 1 kg each, are selected, the imperfect (black, adjoined, shell, badly grained, bored, broken, coconut, sailor), as well as all the impurities (sticks, stones, lumps and husks) are excluded.

### 2. Grinding, extraction and isomerization

The grains selected as raw material are ground, until a granulation under 0,8 mm is obtained. The powder obtained is separated in several different samples, obtained by mixing hot water in different temperatures, 60°, 80° and 100°C, for 5, 10 and 15 minutes, followed by filtration.

Samples are solved in 80 mL of methanol aqueous solution (40%), and transferred to a 100 ml container. The Carrez solution (2 mL of each component) is added to the mixture and put to decant for 10 minutes. The precipitate is removed by gravitational filtering with the use of a Whatman N/1 filter paper, and the filtrate is used for chromatographic analysis.

The chlorogenic acids isomerization is made by adding 5-cafeoilquinic acid (200 mg) solved in distilled water and the pH adjusted to 8 with ammonia solution. The solution is heated for 30 minutes in boiling water (100°C). The pH is then adjusted to 2.5 to 3.0 with hydrochloric acid after cooling up to room temperature. For the feruloilquinic acid isomers preparation, 20 µL of 5-feruloilquinic acid standard solution are added to 5 mL of water adjusted to pH 8.

Then, it is heated in boiling water for 15 minutes, followed by evaporation until drying and analysis.

### 3. Quantitative analysis

Each sample is submitted to a chromatographic analysis, with the use of a specific methodology, described as follows:

### 3.1 Reagents

Carrez Solution (clearer agent): solution I is prepared by dissolution of 21.9 g of crystallized zinc acetate [Zn (C₂H₃O₃) 2.2 H₂O] and 3 mL of glacial acetic acid in distilled water up to 100 mL; solution II consists of 10.6 g of potassium hexaferrate in 100 mL of distilled water.

4 M ammonia solution

4 M hydrochloric acid

Solvents for extraction: Methanol / water (40% / 70% v/v); Propyl Alcohol / water (70% v/v).

Solvents for chromatography: 0.01 M Tripotassium citrate adjusted to pH 2.5 with hydrochloric acid and methanol for H.P.L.C.

Chlorogenic acid solutions: 5-cafeoilquinic acid, Sigma - England; 5-feruloilquinic acid 1 mg/1mL, Surrey - England; Trigonelline, Sigma - England.

### 3.2 Device and chromatographic conditions

A SHIMADZU (Japan) High Performance Liquid Chromatography (H.P.L.C.) device with a pump and an ultraviolet detector (UV, 0.16 AUFS at 272 nm), and a RHEODYNE injection valve with a 20 µL fixed loop. A pre-column (50 X 5 mm) with silica (7 - 150 µ) is placed before the injection valve to saturate the moving phase.

A TSK G-3000 SW HPGF (300 X 8 mm) chromatographic column and the respective holder (SUPELCO, USA) are used. The moving phase consists of bidistilled water in a flow of 0.5 ml/min. The quantification is made by comparison of the caffeine, 5-cafeoilquinic acid, trigonelline and 5-feruloilquinic acid standards peaks.

### 4. Mechanical and chemical preparation:

The samples in which analytical detection is made, of a caffeine content within the range from 80 to 90 mg, and of chlorogenic acids within the range from 250 to 450 mg per 1200 mg of dry extract are, as in Example 1, selected for encapsulation (capsules with 1 g from the sample), and preparation of a suspension, as in Example 2. These (the positive samples) will show, on the average, the following composition per gram:

| SUBSTANCES | CONTENT |
|---|---|
| Caffeine | 80 mg/g |
| 5-p-cumaroilquinic acid | 180 mg/g |
| Feruloilquinic acid | 20 mg/g |
| Dicafeoilquinic acid | 70 mg/g |
| Cafeoilquinic acid | 25 mg/g |
| Cafeoilferuloilquinic acid | 25 mg/g |
| Amino Acids | traces |
| Mineral Salts | traces |
| Sugars | traces |
| Lipids | traces |
| Others (ashes, pigments, oils, cafestol, etc.) | non-existent |

It is then, observed, that concerning the natural composition of coffee (green or roasted), the product composition object of this patent shows different contents for all its constituents, emphasizing the caffeine and chlorogenic acids contents (about 8% and 32%, respectively), substances responsible for the actions of the product on the prophylaxis of depression and alcoholism.

From the positive samples, through simple encapsulation and suspensions formation processes, the products are obtained as specified in the following Examples 1 and 2:

### Example 1

Each capsule (1 g) contains:

| | |
|---|---|
| Caffeine | 80 mg |
| 5-p-coumaroilquinic acid | 180 mg |
| Feruloilquinic acid | 20 mg |
| Dicafeoilquinic acid | 70 mg |
| Cafeoilquinic acid | 25 mg |
| Cafeoilferuloilquinic acid | 25 mg |
| Excipient q.s.p | 1 capsule |

### Example 2

Each 5 mL (1 teaspoonful) of the suspension contains:

| | |
|---|---|
| Caffeine | 80 mg |
| 5-p-coumaroilquinic acid | 180 mg |
| Feruloilquinic acid | 20 mg |
| Dicafeoilquinic acid | 70 mg |
| Cafeoilquinic acid | 25 mg |
| Cafeoilferuloilquinic acid | 25 mg |
| Vehicle q.s.p | 5 mL |

The recommended dosage of the product object of this patent of invention for the prophylaxis of depression and alcoholism, in young people and adults, is one capsule or one teaspoonful ingested in the morning, daily.

As it can be understood, the present invention fills a very important gap in the state of the technique, which does not comprise the registration of a product with the same purpose of this Extract, with antidepressant and opioid antagonist properties, which may thus come to assume an incalculably important part for our Country public health and economy

## Claims

1. A green coffee extract having the following composition:
- Caffeine 80mg/g;
- 5-p-cumaroylquinic acid 180 mg/g;
- Feruloylquinic acid 20 mg/g;
- Dicaffeoylquinic acid 70 mg/g;
- Caffeoylquinic acid 25 mg/g;
- Caffeoylferuloylquinic acid 25 mg/g.

2. The use of an extract of claim 1 for the preparation of a medicament for the prophylaxis of depression and alcoholism.

3. A pharmaceutical composition comprising as the active ingredient the extract of claim 1.

4. A process for the preparation of the extract of claim 1 which comprises the extraction of grounded green coffee grains with water at 60, 80 or 100°C for 5, 10 or 15 minutes followed by filtration, addition of a 40% methanol aqueous solution to which a solution of zinc acetate in acetic acid is added.

## Patentansprüche

1. Grüner Kaffee-Extrakt mit der folgenden Zusammensetzung:
- Koffein 80 mg/g;
- 5-p-Cumaroylchinasäure 180 mg/g;
- Feruloylchinasäure 20 mg/g;
- Dicaffeoylchinasäure 70 mg/g;
- Caffeoylchinasäure 25 mg/g;
- Caffeoylferuloylchinasäure 25 mg/g.

2. Verwendung eines Extraktes nach Anspruch 1 für die Herstellung eines Medikaments für die Vorbeugung vor Depression und Alkoholismus.

3. Pharmazeutische Zusammensetzung, die als aktiven Inhaltstoff den Extrakt nach Anspruch 1 enthält.

4. Verfahren für die Herstellung des Extraktes nach Anspruch 1, das die Extraktion von gemahlenen, grünen Kaffeebohnen mit Wasser bei 60 °C, 80 °C oder 100 °C für 5, 10 oder 15 Minuten, anschließender Filtration, Zugabe von einer wässrigen 40%igen Methanollösung, zu der eine Lösung von Zinkacetat in Essigsäure zugefügt ist, umfasst.

## Revendications

1. Extrait de café vert ayant la composition suivante :
- caféine 80 mg/g ;
- acide 5-p-cumaroylquinique 180 mg/g ;
- acide féruloylquinique 20 mg/g ;
- acide dicaféoylquinique 70 mg/g ;
- acide caféoylquinique 25 mg/g ;
- acide caféoylféruloylquinique 25 mg/g.

2. Utilisation d'un extrait selon la revendication 1 pour la préparation d'un médicament pour la prophylaxie de la dépression et de l'alcoolisme.

3. Composition pharmaceutique comprenant en tant qu'ingrédient actif l'extrait selon la revendication 1.

4. Procédé pour préparer un extrait selon la revendication 1 qui comprend l'extraction de grains de café vert moulus avec de l'eau à 60, 80 ou 100°C pendant 5, 10 ou 15 minutes suivie d'une filtration, ajout d'une solution aqueuse de méthanol à 40% à laquelle une solution d'acétate de zinc dans de l'acide acétique est ajoutée.
